# EUROPEAN PATENT SPECIFICATION

(11) **EP 2 900 253 B1**
(45) Date of publication and mention of the grant of the patent: **14.11.2018**
(21) Application number: 13841917.1
(22) Date of filing: 27.09.2013
(51) Int. Cl.: A61K 38/00, A61P 25/00, C12N 9/02, A61K 38/44, A61K 38/17

(54) **METHODS FOR ALLEVIATING SYMPTOMS OF MULTIPLE SCLEROSIS BASED ON APOAEQUORIN-CONTAINING COMPOSITIONS**
VERFAHREN ZUR LINDERUNG VON SYMPTOMEN VON MULTIPLER SKLEROSE AUF DER BASIS VON APOAEQUORINHALTIGEN ZUSAMMENSETZUNGEN
PROCÉDÉS D'ATTÉNUATION DES SYMPTÔMES DE LA SCLÉROSE EN PLAQUES BASÉS SUR DES COMPOSITIONS CONTENANT DE L'APOAEQUORINE

(30) Priority: 27.09.2012 US 201261706615 P
(43) Date of publication of application: 05.08.2015
(73) Proprietor: Quincy Bioscience, LLC, Madison, WI 53717 (US)
(72) Inventor: UNDERWOOD, Mark, Y., Madison, WI 53717 (US)
(74) Representative: advotec.
(86) International application number: PCT/US2013/062268
(87) International publication number: WO 2014/052807

(56) References cited:
- WO-A1-2012/022740
- US-A1- 2008 293 092
- US-A1- 2011 124 562
- US-A1- 2011 130 336
- 'Quincy bioscience puts hope in walk for multiple sclerosis' PRWEB NEWSWIRE 29 April 2011, XP055249239 Retrieved from the Internet: <URL:http://search.proquest.com/professiona l/docview/864500341?accountid=157282>

## Description

### FIELD OF THE INVENTION

This invention relates generally to the method of using apoaequorin-containing compositions for alleviating symptoms associated with Multiple Sclerosis.

### BACKGROUND OF THE INVENTION

Multiple sclerosis (MS) is an autoimmune disease that affects the brain and spinal cord. In MS, an individual's immune system attacks the myelin proteins protecting and insulating the nerve cells. This inappropriate immune attack on "self' cells results in inflammation, damage, and death of these cells which in turn results in distorted, slowed or stopped signaling of that nerve. This interruption of nerve signaling has profound and widespread effects on an individual's health and well-being; the body and brain no longer communicate effectively, causing problems that can appear throughout the body.

MS affects approximately 400,000 people in the United States alone (National Multiple Sclerosis Society). MS is typically diagnosed between the ages of 20 and 40 although it can arise at nearly any age. MS affects both men and women although it does seem to occur more often in women. There is no clear trigger for onset of disease nor is there a clear cause. Treatment for MS is itself unclear in part due to the aforementioned absence of a known cause as well the unpredictable and variable symptoms. The symptoms of MS vary both from person to person and day to day. MS attacks the nerve center resulting in symptoms that range to all part of the body and affect multiple tissue types. For example, symptoms affecting the musculature may include: muscular weakness, numbness, spasms, tremors, pain, loss of balance, or loss of muscle control. Symptoms of nervous system involvement may include: dizziness, fatigue, hearing and vision loss, or double vision. There are combined symptoms including trouble breathing and swallowing, uncontrolled eye movement, trouble chewing and speaking. Other symptoms are more directly related to brain function such as decreased attention span, poor judgment, memory loss, difficulty reasoning and solving problems, and/or depression. MS not only wrests control of your body from you, but also takes your mind, your ability to think and communicate clearly.

MS is a disease that is partially managed but not cured. While there is currently no cure for MS, there are several therapies that may slow progression of the disease or reduce the frequency of relapse. The goal of treatment is to control symptoms and help maintain quality of life in individuals with MS. Individuals with MS must take several therapies to manage their disease and symptoms. To maintain quality of life some individuals with MS use "disease modifying drugs" (DMDs). It is currently suggested that a diagnosed individual start use of these drugs immediately. DMDs used to slow progression of MS include a variety of immune modulators and immunosuppressants and are taken on a long-term basis. There is some evidence that these drugs lessen severity and frequency of MS attacks as well as reduce accumulation of lesions in the brain, which may slow progression of disease. DMDs therapies have side effects ranging from febrile illness to liver damage, and do not halt disease progression or cure the disease.

As MS progresses so do the symptoms and the severity of these symptoms. When symptoms are not ameliorated by DMDs, additional medications may be taken to manage these symptoms. Physical symptoms can be partially ameliorated through physical, speech, and psychological therapies, in addition to the battery of drugs specific for the symptoms an individual experiences. Regimens are tailored to individual patients and change over time.

US 2011/124562 A1 discloses compositions containing apoaequorin for use in treating symptoms and disorders related to calcium imbalances. Said document discloses that a disruption in calcium homeostasis is associated with many diseases, syndromes and conditions.

"Quincy bioscience puts hope in walk for multiple sclerosis", PRWEB NEWSWIRE, 29 April 2011, XP055249239, discloses that apoaequorin may improve the quality of life for those with multiple sclerosis.

What is needed in the art is a method of combating cognitive degradation and protecting mental facilities from the ravages of the immune systems attack on neurons. The art also needs a therapy that broadly alleviates the symptoms of MS especially those symptoms related to fatigue, improving quality of life for MS patients overall.

### SUMMARY OF THE INVENTION

The present invention is directed to apoaequorin for alleviating a symptom of MS, comprising administering to a subject in need of such treatment an effective amount of apoaequorin.

Apoaequorin according to the invention is useful in treating a wide variety of symptoms associated with multiple sclerosis, including but not limited to disruptions in sleep quality, energy quality, mood quality, pain, and/or cognitive functioning. In methods, apoaequorin is preferably administered to the subject in the form of a pharmaceutical composition.

Other objects, features and advantages of the present invention will become apparent after review of the specification and claims.

### BRIEF DESCRIPTION OF THE DRAWINGS

Figure 1 is a chart showing the differences of the Multiple Sclerosis Impact Scale (MSIS) scores between the apoaequorin arm and the placebo arm groups.
Figure 2 is a chart showing the differences of the Physical Health subscale of the Multiple Sclerosis Quality of Life (MSQOL) scores between the apoaequorin arm and the placebo arm groups.
Figure 3 is a chart showing the differences of the Physical Health subscale of the MSQOL between the apoaequorin and the placebo groups.
Figure 4 is a chart showing the differences of the Role Limitations-Physical subscale of the MSQOL between the apoaequorin and the placebo groups.
Figure 5 is a chart showing the differences of the Role Limitations-Emotional subscale of the MSQOL between the apoaequorin and the placebo groups.
Figure 6 is a chart showing the differences of the Pain subscale of the MSQOL between the apoaequorin and the placebo groups.
Figure 7 is a chart showing the differences of the Modified Fatigue Impact Scale (MFIS) composite scores between the apoaequorin and the placebo groups.
Figure 8 is a chart showing the differences of the MFIS Physical subscale composite scores between the apoaequorin and the placebo groups.
Figure 9 is a chart showing the differences of the MFIS Cognitive subscale composite scores between the apoaequorin and the placebo groups.
Figure 10 is a chart showing the differences of the MSQOL Physical Health composite scores between the apoaequorin and the placebo groups.
Figure 11 is a chart showing the differences of the MSQOL Mental Health composite scores between the apoaequorin and the placebo groups.
Figure 12 is a chart showing the differences of the MSQOL Role Limitations-Physical subscale between the apoaequorin and the placebo groups.
Figure 13 is a chart showing the differences of the MSQOL Cognitive subscale between the apoaequorin and the placebo groups.
Figure 14 is a chart showing the differences of the Multiple Sclerosis Impact Scale (MSIS) scores between the apoaequorin and the placebo groups.
Figure 15 is a chart showing the differences of the MFIS Composite scores between the apoaequorin and the placebo groups.
Figure 16 is a chart showing the differences of the MFIS Physical Health subscale between the apoaequorin and the placebo groups.
Figure 17 is a chart showing the differences of the MFIS Cognitive subscale between the apoaequorin and the placebo groups.
Figure 18 is a chart showing the differences of the MFIS Psychosocial subscale between the apoaequorin and the placebo groups.
Figure 19 is a chart showing the differences of the MSQOL Physical Health Composite scores between the apoaequorin and the placebo groups.
Figure 20 is a chart showing the differences of the MSQOL Physical Health subscale between the apoaequorin and the placebo groups.
Figure 21 is a chart showing the differences of the MSQOL Role Limitations-Physical subscale between the apoaequorin and the placebo groups.
Figure 22 is a chart showing the differences of the MSQOL Pain subscale between the apoaequorin and the placebo groups.
Figure 23 is a chart showing the differences of the MSQOL Social Function subscale between the apoaequorin and the placebo groups.

### DETAILED DESCRIPTION OF THE INVENTION

### I. IN GENERAL

Before the present materials and methods are described, it is understood that this invention is not limited to the particular methodology, and materials described, as these may vary. It is also to be understood that the terminology used herein is for the purpose of describing particular embodiments only, and is not intended to limit the scope of the present invention which will be limited only by the appended claims.

It must be noted that as used herein and in the appended claims, the singular forms "a", "an", and "the" include plural reference unless the context clearly dictates otherwise. As well, the terms "a" (or "an"), "one or more" and "at least one" can be used interchangeably herein. It is also to be noted that the terms "comprising", "including", and "having" can be used interchangeably.

Unless defined otherwise, all technical and scientific terms used herein have the same meanings as commonly understood by one of ordinary skill in the art to which this invention belongs. Although any methods and materials similar or equivalent to those described herein can be used in the practice or testing of the present invention, the preferred methods and materials are now described. All publications and patents specifically mentioned herein are incorporated by reference for all purposes including describing and disclosing the chemicals, instruments, statistical analysis and methodologies which are reported in the publications which might be used in connection with the invention. All references cited in this specification are to be taken as indicative of the level of skill in the art. Nothing herein is to be construed as an admission that the invention is not entitled to antedate such disclosure by virtue of prior invention.

### II. THE INVENTION

Apoaequorin is classified as a "calcium-binding protein" which is necessary for the process of calcium regulation in nerve cells. Apoaequorin has proven neuroprotective capabilities, protecting cells from the damage of calcium overload. Calcium is a critical part of cell communication. Without calcium providing the "electricity" to the cell, we wouldn't be able to think, move, or experience emotion. Excess calcium can cause overstimulation of the cell and trigger other mechanisms, which lead to a break down in cell function. Research on the effects of apoaequorin on the brain and body have shown promise for enhancing and improving memory, improving sleep quality, increasing brain cell survival and improving cognitive function.

The present invention is directed to the administration of apoaequorin-containing compositions to a subject in order to alleviate a range of the symptoms experienced by individuals suffering from MS. The maintenance of ionic calcium concentrations in plasma and body fluids is understood to be critical to a wide variety of bodily functions, including, but not limited to neuronal excitability, muscle contraction, poor sleep quality, low energy quality, poor mood quality, disruption of memory and pain.

In certain applications apoaequorin is administered alone or in combination with disease modifying drugs ("DMDs") for reducing the recurrence of symptoms or alleviating the symptoms of Multiple Sclerosis. Further, methods are described, which comprise administering apoaequorin in combination with one or more additional agents having known therapeutic or nutraceutical value. Particularly preferred applications of apoaequorin are in treating one or more well-known symptoms and disorders related to MS such as quality of sleep, energy, mood, cognitive function, or pain.

As used herein, the term "treating" includes preventative as well as disorder remittent treatment. As used herein, the terms "reducing", "alleviating", "ameliorating" "suppressing" and "inhibiting" have their commonly understood meaning of lessening or decreasing. As used herein, the term "progression" means increasing in scope or severity, advancing, growing or becoming worse. As used herein, the term "recurrence" means the return of a disease after a remission.

As used herein, the term "administering" refers to bringing a patient in contact with apoaequorin. In preferred embodiments, the present invention encompasses administering the compositions useful in the present invention to a patient or subject. A "patient" or "subject", used equivalently herein, refers to a human that has been diagnosed with multiple sclerosis, preferably diagnosed with either the RRMS or SPMS forms of MS.

As used herein, the terms "effective amount" and "therapeutically effective amount" refer to the quantity of active agents sufficient to yield a desired therapeutic response without undue adverse side effects such as toxicity, irritation, or allergic response. The specific "effective amount" will, obviously, vary with such factors as the stage of the disease, whether the individual is in remission or active MS, the particular symptoms being treated, the physical condition of the patient, the duration of the treatment, the nature of concurrent therapy (if any), and the specific formulations employed and the structure of the compounds or its derivatives. In this case, an amount would be deemed therapeutically effective if it resulted in one or more of the following: (1) alleviation or amelioration of symptoms as measured by standard tests and questionnaires, (2) stabilization of disease/ maintenance of a remission like state, (3) improved quality of life as determined by the individual, (4) reduction in severity and/or occurrence of symptoms. The optimum effective amounts can be readily determined by one of ordinary skill in the art using routine experimentation.

According to the invention, apoaequorin is formulated with at least one acceptable carrier at a dosage of approximately 40-80 mg/dose, in either a single dose (i.e., one capsule per day preferably in the morning hours) or in multiple doses (i.e., split into several equal or unequal doses during a 24 hour time period) taken every day.

Apoaequorin-containing compositions described herein may be provided in the form of pharmaceutical compositions where apoaequorin prevents the onset of, reduces the occurrence or duration of, or stabilizes various symptoms related to MS. A pharmaceutical composition according to the present invention may contain only apoaequorin as an active ingredient, or alternatively, may further comprise, in admixture with dietary supplements including vitamins, co-enzymes, minerals, herbs, amino acids and the like which supplement the diet by increasing the total intake of that substance.

Therefore, the present application describes methods of providing pharmaceutical benefits to a patient comprising the step of administering to the patient a pharmaceutical composition containing apoaequorin. Such compositions generally include a "pharmaceutically-acceptable carrier" which, as referred to herein, is any carrier suitable for delivery, preferably oral delivery, including aforementioned pharmaceutically acceptable carriers. In certain embodiments, pharmaceutical compositions according to the invention further comprise dietary supplements, which, defined on a functional basis, include immune boosting agents, anti-inflammatory agents, anti-oxidant agents, anti-viral agents, or mixtures thereof.

The invention will be more fully understood upon consideration of the following non-limiting Examples.

### EXAMPLES

Three different qualitative assessment instruments or tools were used to examine the effect of apoaequorin on Multiple Sclerosis. These were the Multiple Sclerosis Impact Scale (MSIS), the Modified Fatigue Impact Scale (MFIS), and Multiple Sclerosis Quality of Life (MSQOL). The dosage of apoaequorin utilized throughout these studies was 40 mg/day administered orally, unless indicated otherwise.

The Multiple Sclerosis Impact Scale (MSIS) is a 29 item, self-report scale that measures the physical and psychological impact of MS from the patient's perspective. The MSIS is widely used to measure therapeutic outcomes in patients with MS.

The Modified Fatigue Impact Scale is a modified form of the Fatigue Impact Scale (Fisk et al, 1994b) based on items derived from interviews with MS patients concerning how fatigue affects their lives. Fatigue is a common and frequently disabling symptom of MS. In individuals with MS, fatigue can significantly impair the ability to function in day-to-day activities. The MFIS focuses on the ways in which MS-related fatigue affects everyday life. The MFIS consists of 21 items on a Likert scale. The MFIS has the three subscales: Physical, Cognitive, and Psychosocial functioning. These subscales examine the effect of particular interventions on fatigue as it relates to these different functional areas.

The Multiple Sclerosis Quality of Life (MSQOL) is a 54-item multidimensional health-related quality of life measure that combines both generic and MS-specific items. (Vickrey et al, 1995) (Vickrey et al, 1997) This instrument generates a number of different subscales that examine the effect of an individual's MS on different quality of life measures. The MSQOL subscales include: physical function, role limitations-physical, role limitations-emotional, pain, emotional well-being, energy, health perceptions, social function, cognitive function, health distress, overall quality of life, and sexual function. The summary scores are the physical health composite summary and the mental health composite summary.

The population of individuals studied included those with MS of either the Relapsing Remitting Multiple Sclerosis (RRMS) or Secondary Progressive Multiple Sclerosis (SPMS) types. To examine the effect of apoaequorin, this population was stratified several different ways: either RRMS by itself or RRMS and SPMS together; by the presence or absence of Disease Modifying Drugs (DMDs); or disease status at the start of the study: i.e., Remission or Relapse.

While a beneficial effect was seen across all segments of the population, the greatest overall effect is seen in individuals who were in a relapse status prior to beginning apoaequorin. The greatest effect on cognitive function, as measured by the MFIS Cognitive subscale, was seen in those individuals who had RRMS and were in a relapse state. The most significant effect on fatigue, as measured by the MFIS Composite scale, was seen in populations that were also experiencing a relapse. A significant beneficial effect was seen on the MSIS in the population that was not receiving any disease modifying drugs (DMD). This effect was seen in independent of an individual's disease status at the start of apoaequorin administration. Additional positive effects were seen on measures of Physical Health, Role Limitations-Physical, Role Limitations-Emotional, Pain, Emotional Well-Being, Energy, Health Perceptions, Social Function, Cognitive Function, Health Distress, And Overall Quality Of Life.

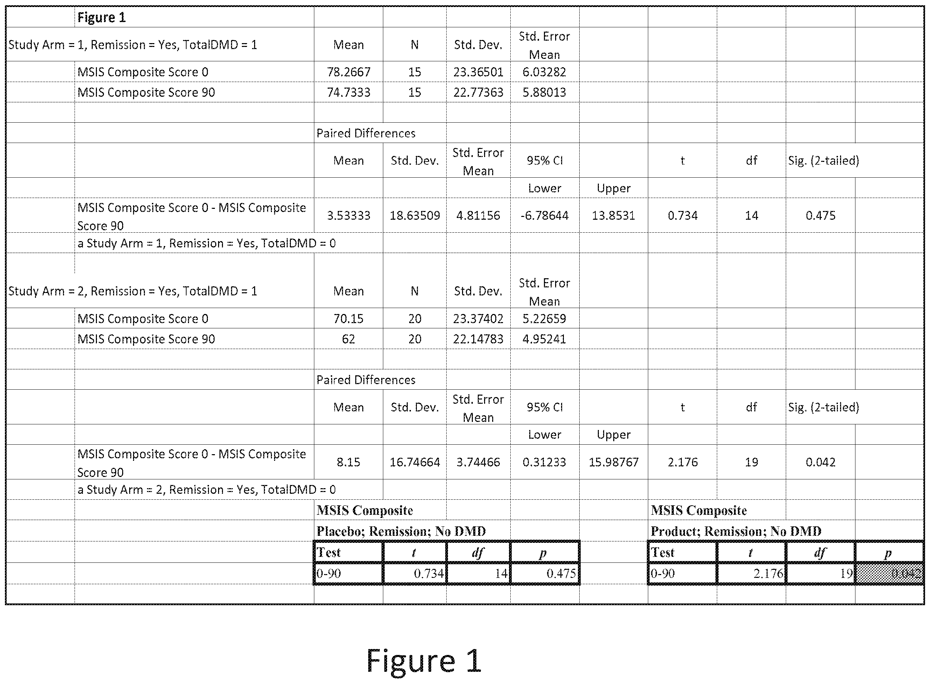

For those individuals with RRMS, who were in remission at the start of the study and were not taking any Disease Modifying Drugs (DMDs), apoaequorin administration showed an 11.6% improvement at Day 90 as compared to Day 0 on the MSIS. The placebo arm showed a 4.51% improvement Day 90 vs. Day 0. The difference between the Day 90 and Day 0 values for the apoaequorin arm were statistically significant (p=<0.05) while the placebo arm did not show significance. (Figure 1).

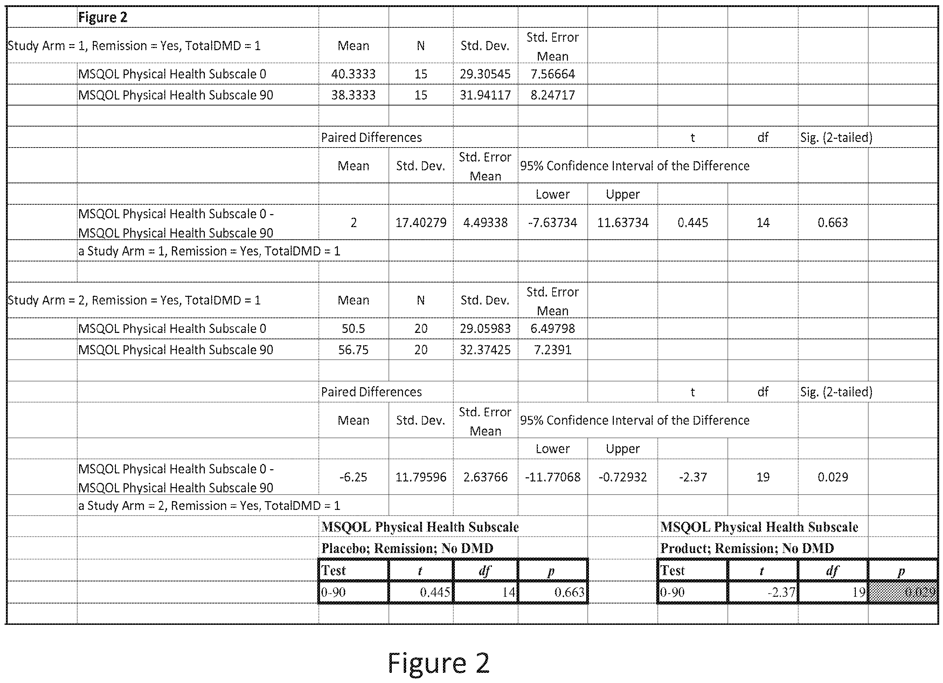

For the aforementioned group, the MSQOL Physical Health subscale scores also showed a statistically significant improvement (Day 90 vs. Day 0) for the apoaequorin arm (p<0.05). No significance was seen in the placebo arm. The apoaequorin arm showed a 12.38% improvement while the placebo reported a 4.96% decrease in scores for the Physical Health subscale of the MSQOL. (Figure 2)

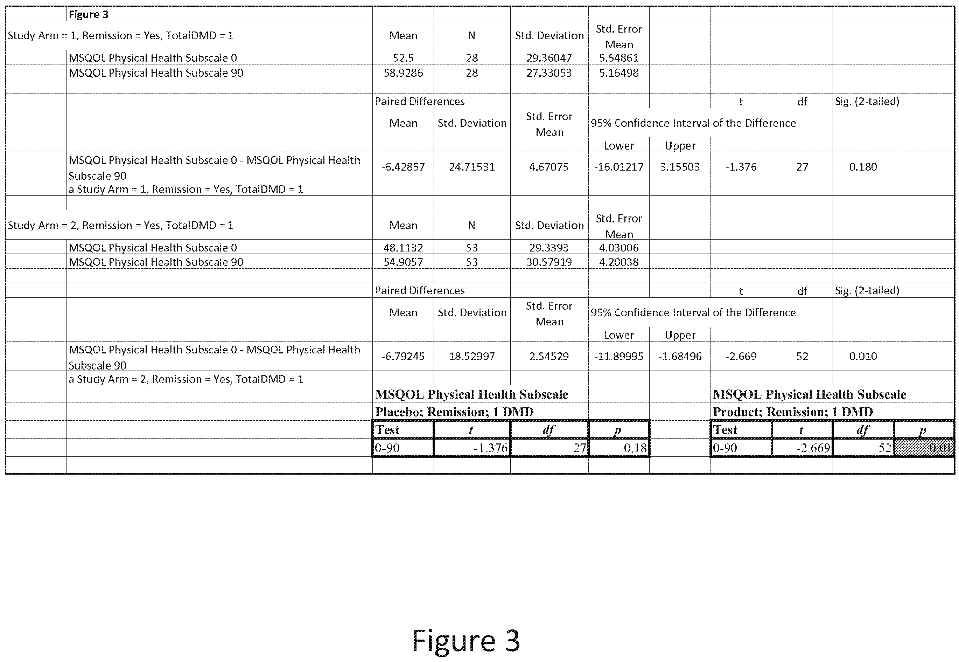

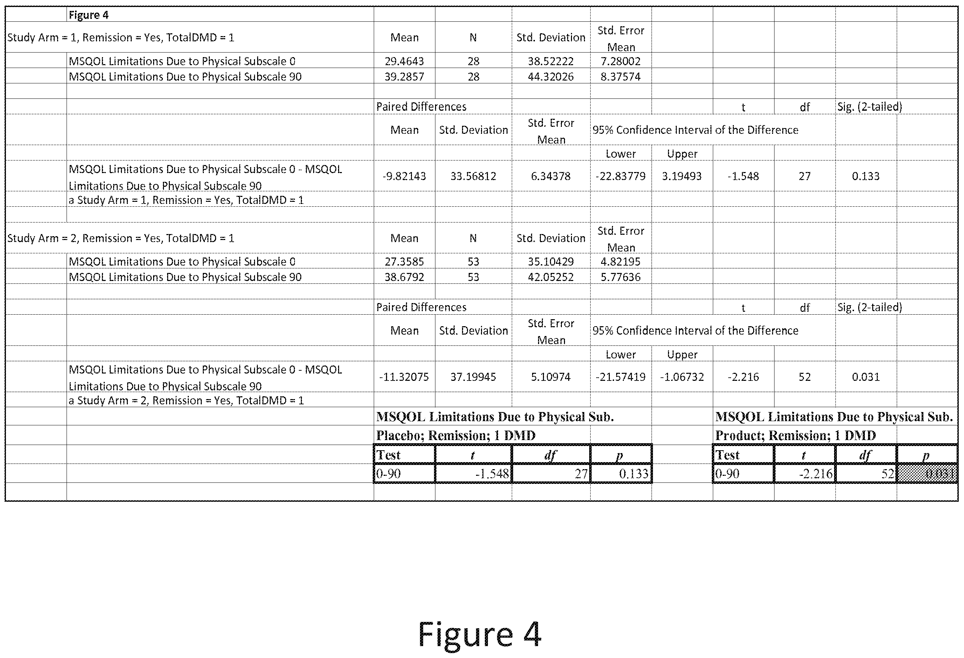

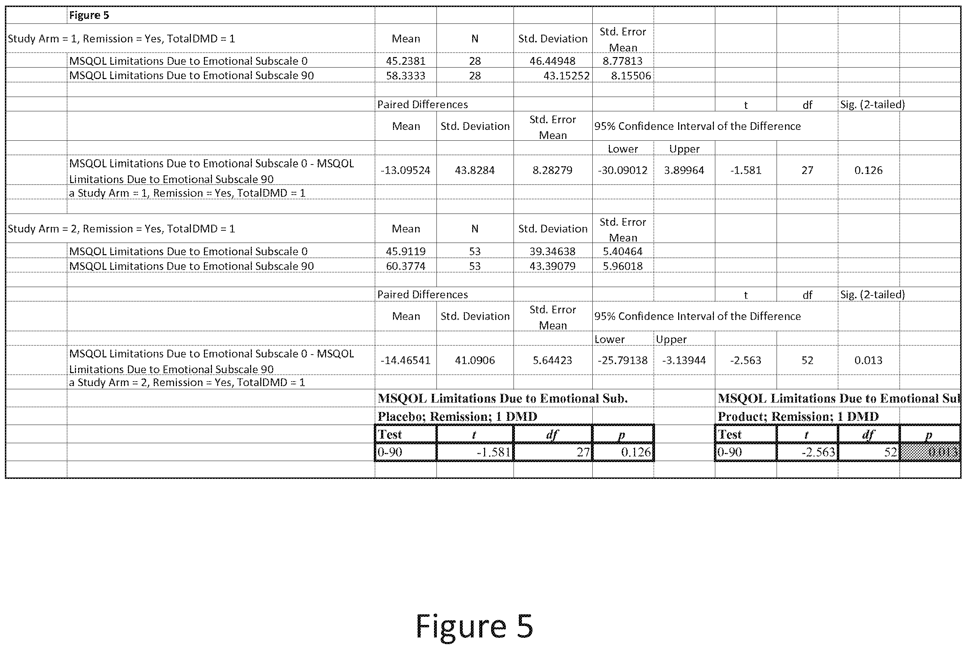

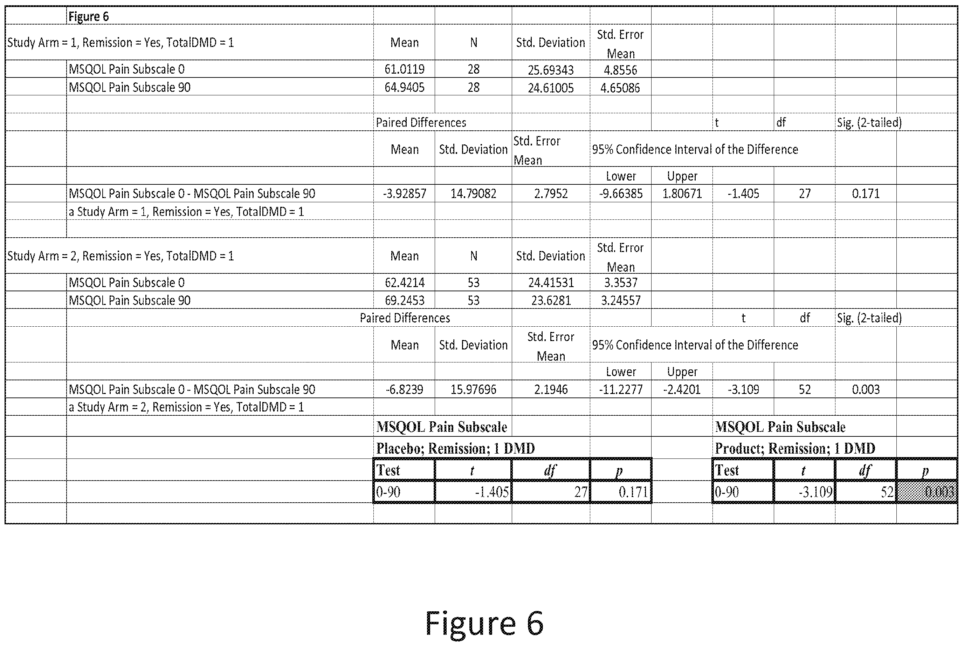

For individuals with RRMS who were in remission at the start of the study and taking a DMD, the apoaequorin arm demonstrated statistically significant improvement (Day 0 vs. Day 90) on several parts of the MSQOL. These include the Physical Health subscale (14.12% for apoaequorin vs. 12.24% for placebo) (Figure 3), Role Limitations-Physical subscale (41.38% for apoaequorin vs. 33.33% for placebo) (Figure 4), Role Limitations-Emotional subscale (31.50% for apoaequorin vs. 28.95% for placebo) (Figure 5), and Pain subscale (10.93% for apoaequorin vs. 6.44% for placebo) (Figure 6). All apoaequorin values are statistically significant to at least p<0.05 while none of the placebo values were statistically significant.

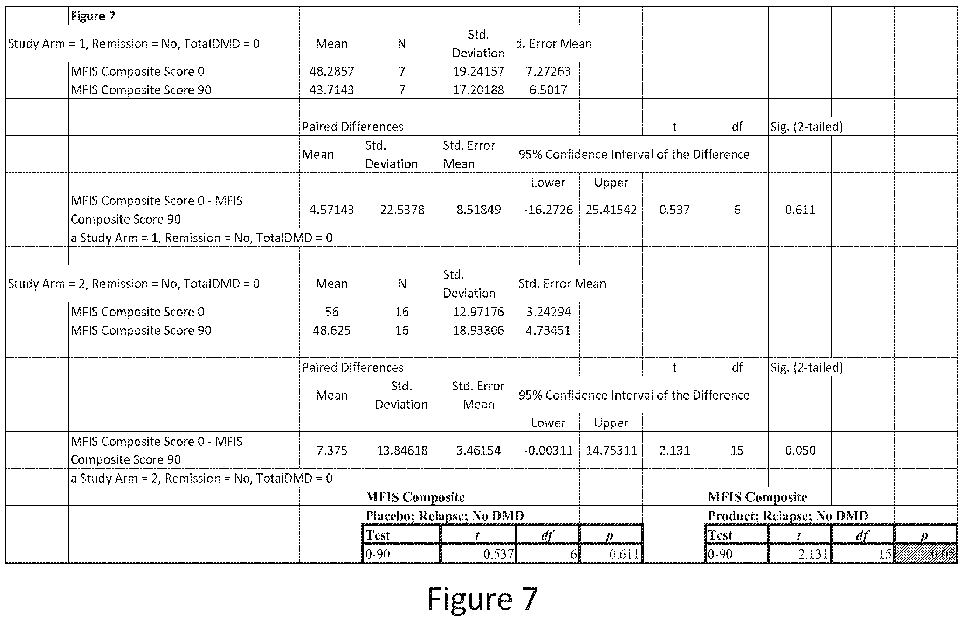

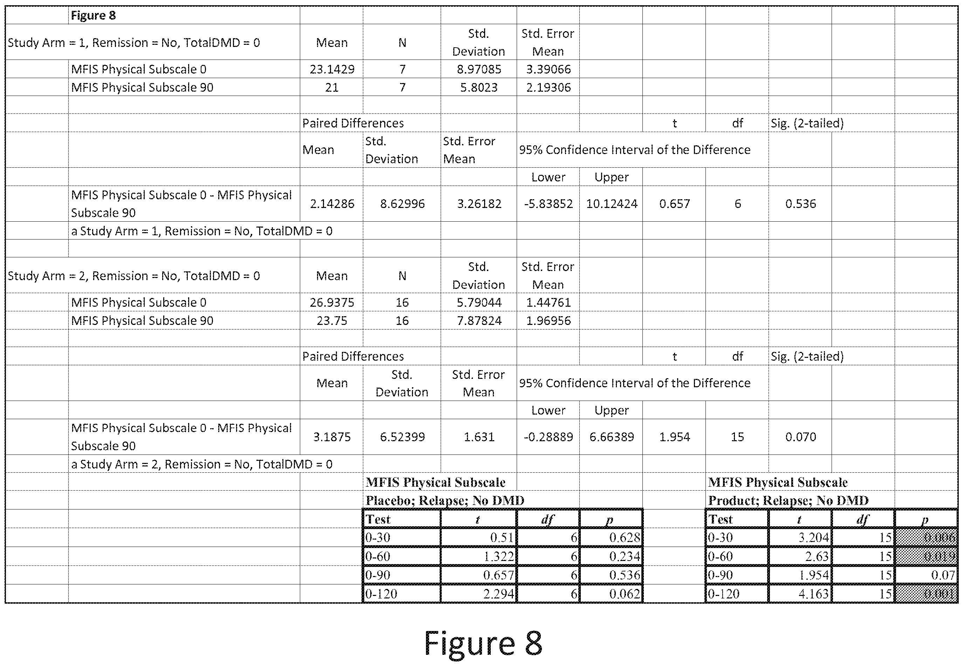

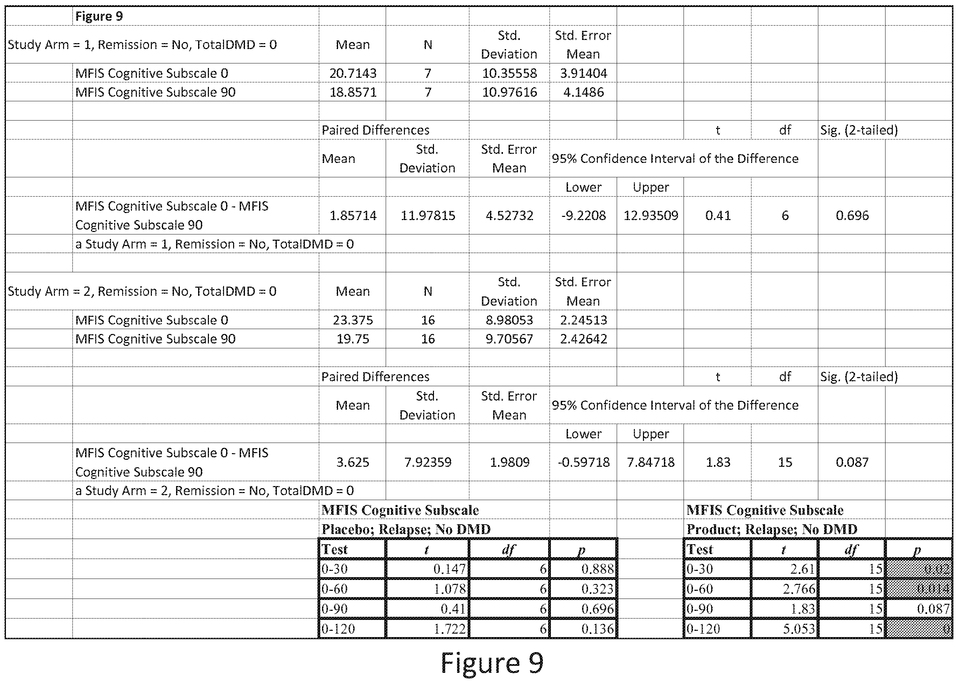

For those individuals with RRMS who were in relapse and not taking a DMD at the beginning of the study, improvements were seen on a number of assessments. The MFIS composite scores improved 13.16% for the apoaequorin arm (Day 90 vs. Day 0) while the placebo arm showed a 9.46% improvement (Figure 7). The MFIS Physical subscale score showed an 11.83% improvement for the apoaequorin arm, while the placebo group showed only a 9.26% improvement comparing Day 90 to Day 0 (Figure 8). The MFIS Cognitive subscale showed a 15.51% improvement for the apoaequorin arm, with the placebo arm reporting an 8.96% improvement (Figure 9). All of the apoaequorin arm values were statistically significant at p=<0.05 or close to statistical significance. None of the aforementioned placebo values showed statistical significance.

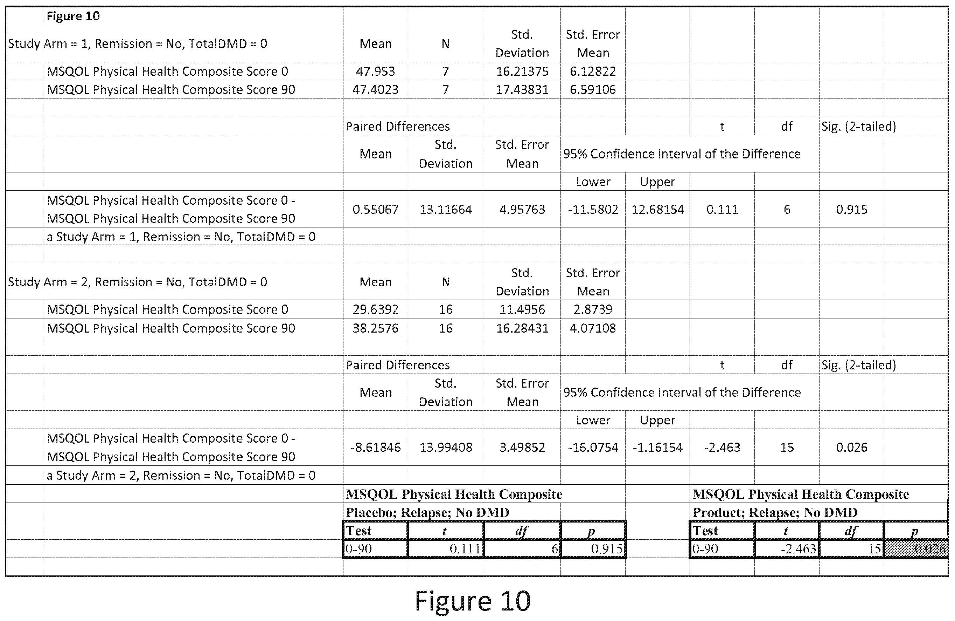

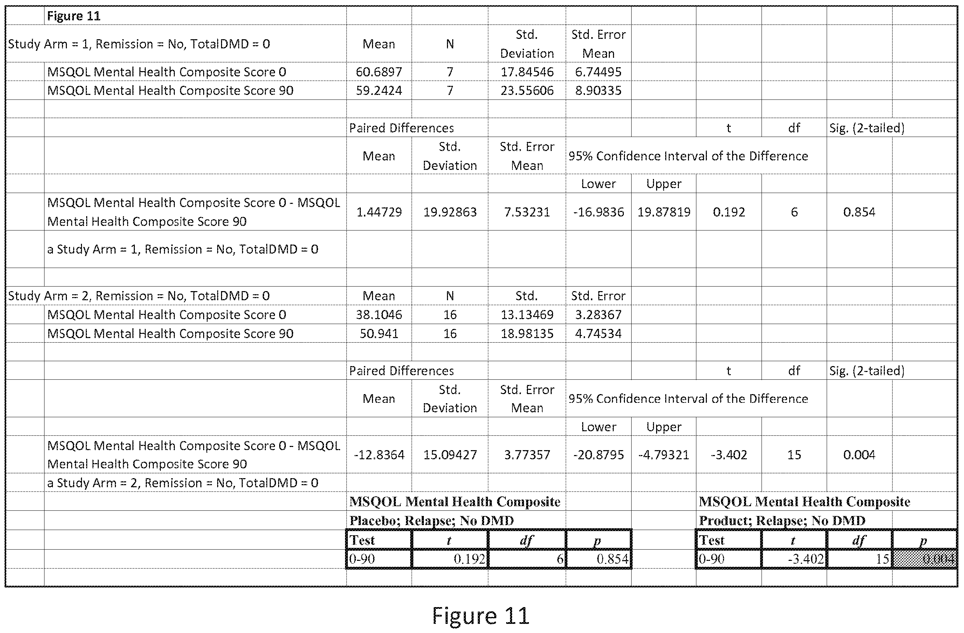

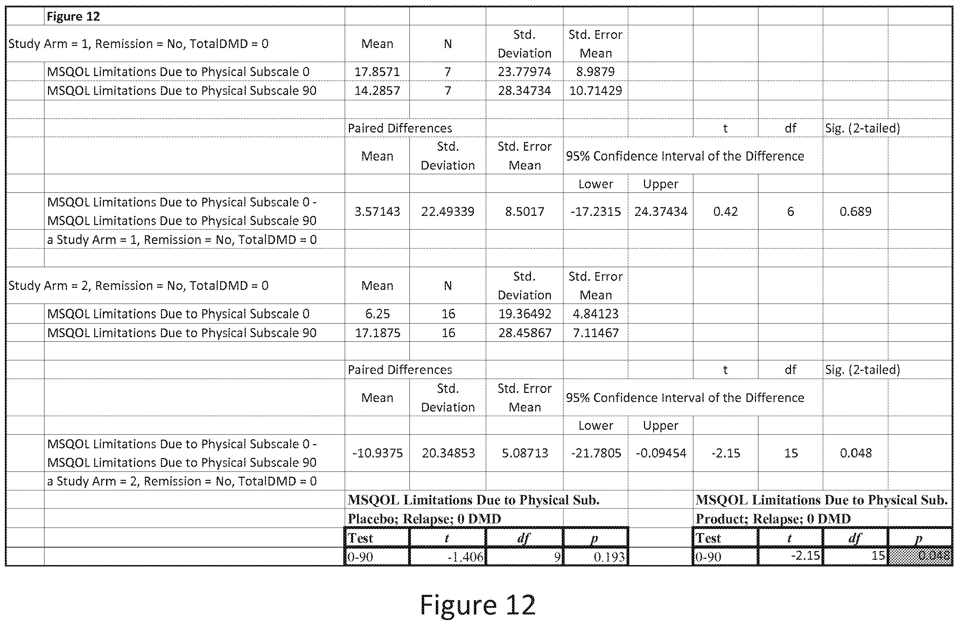

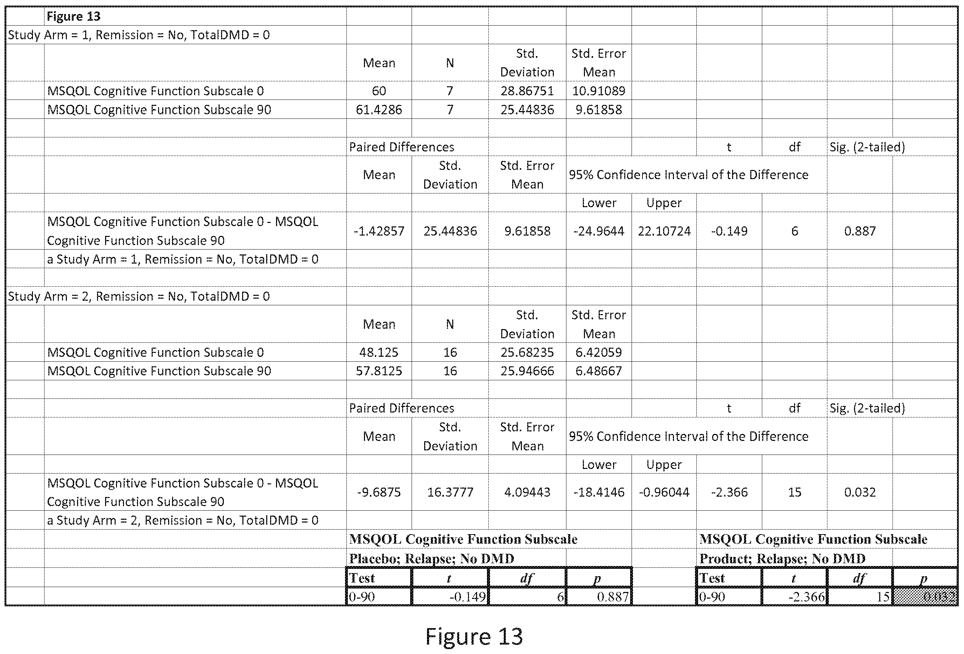

For individuals in the aforementioned group, statistically significant improvements were also seen on the MSQOL Physical Health Composite (29.08% for the apoaequorin arm vs. -1.15% for the placebo arm) (Figure 10), MSQOL Mental Health Composite (33.39% vs. -2.68%) (Figure 11), MSQOL Role Limitations-Physical (175% vs. -20%) (Figure 12), and the MSQOL Cognitive subscale (20.13% vs. 2.38%) (Figure 13).

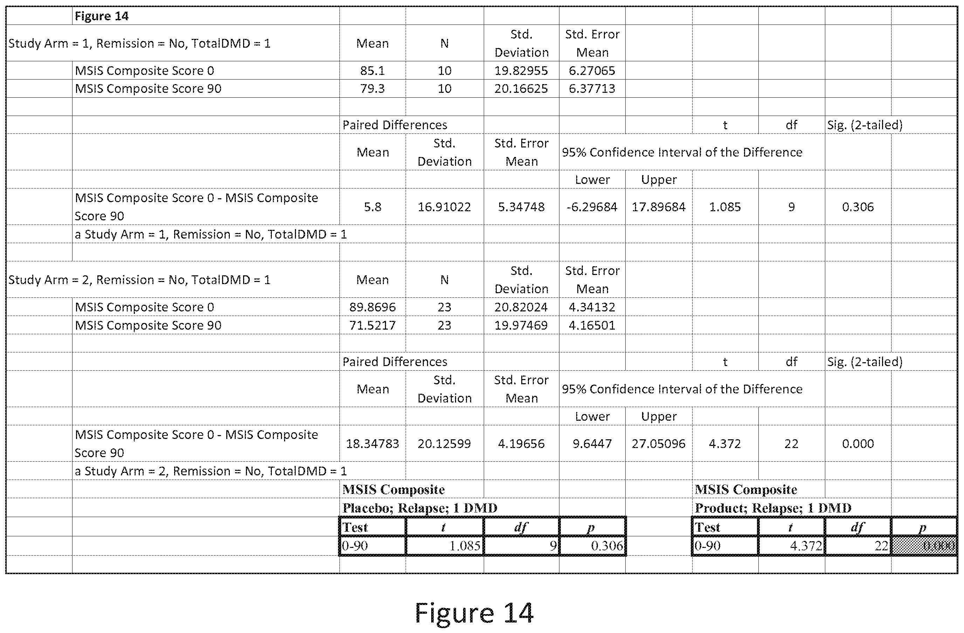

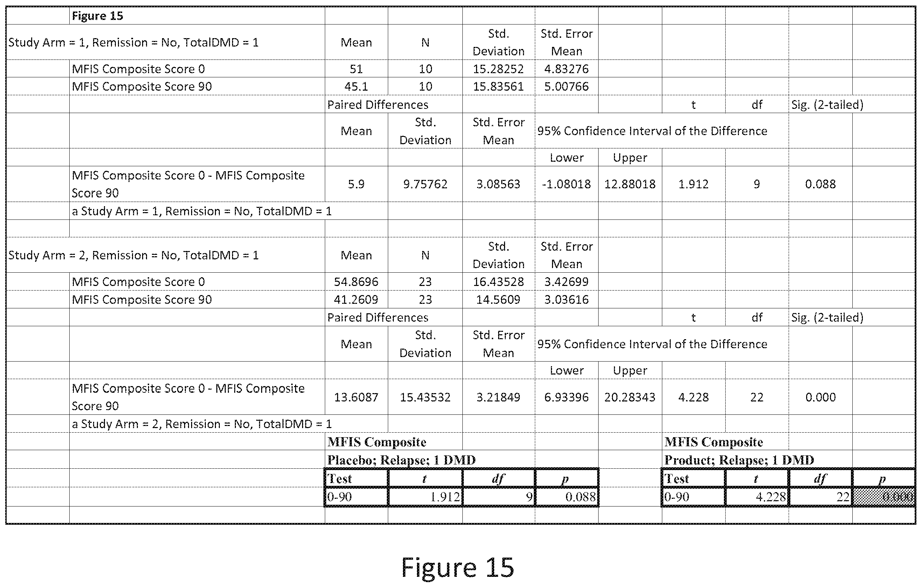

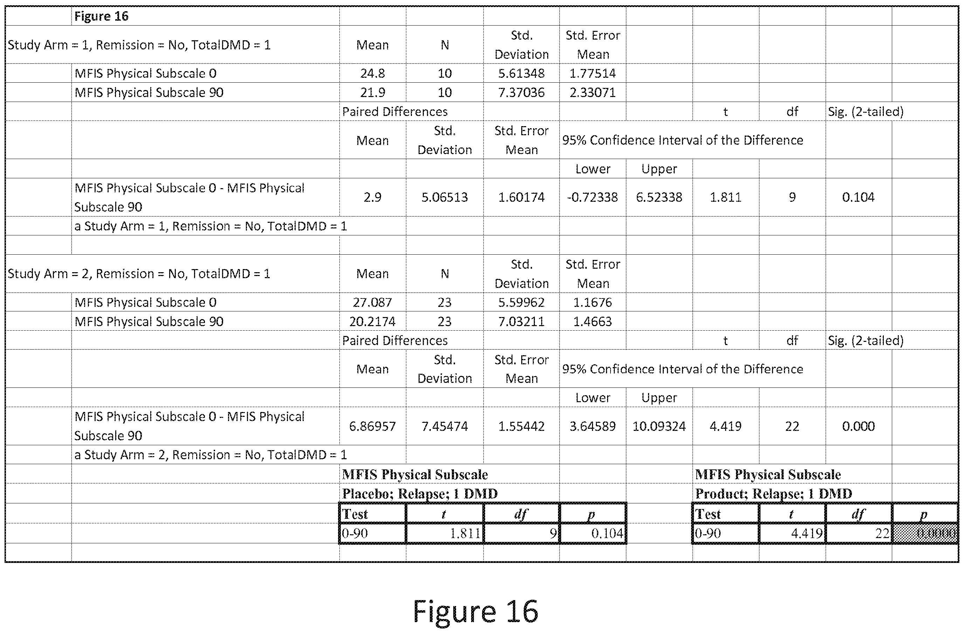

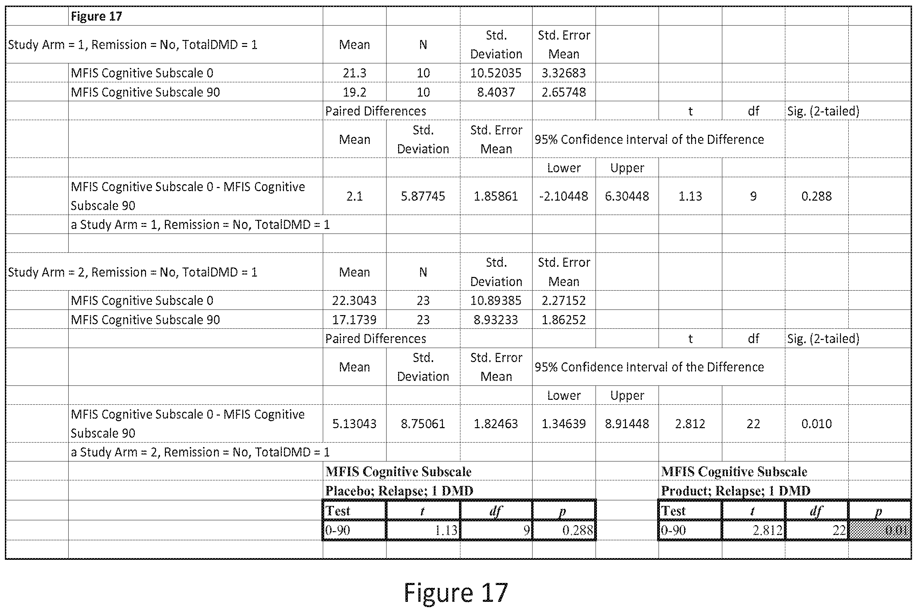

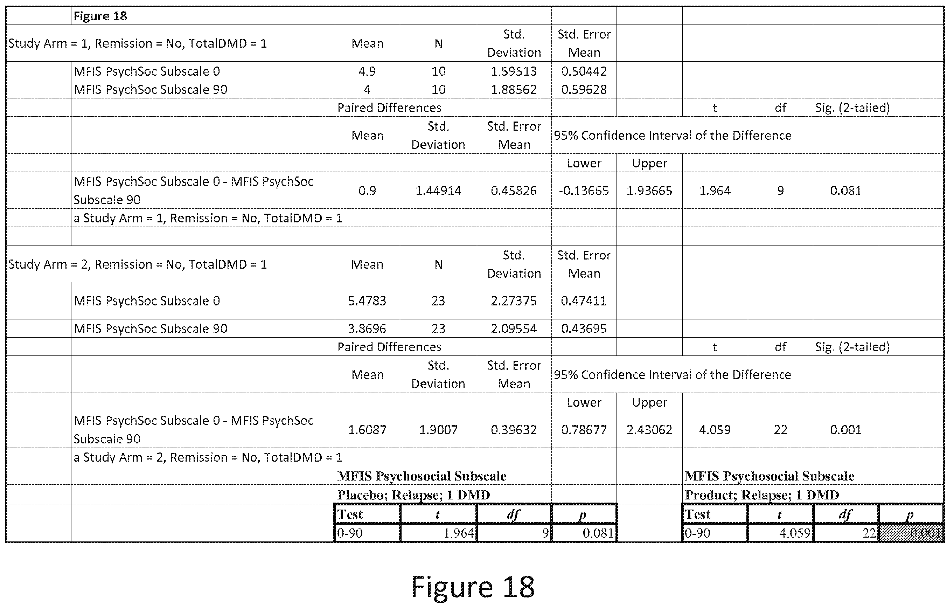

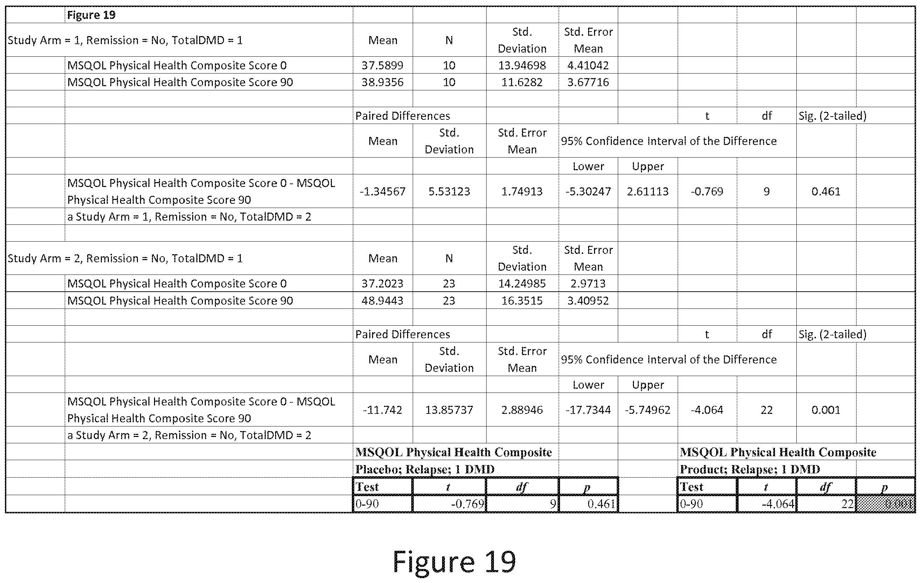

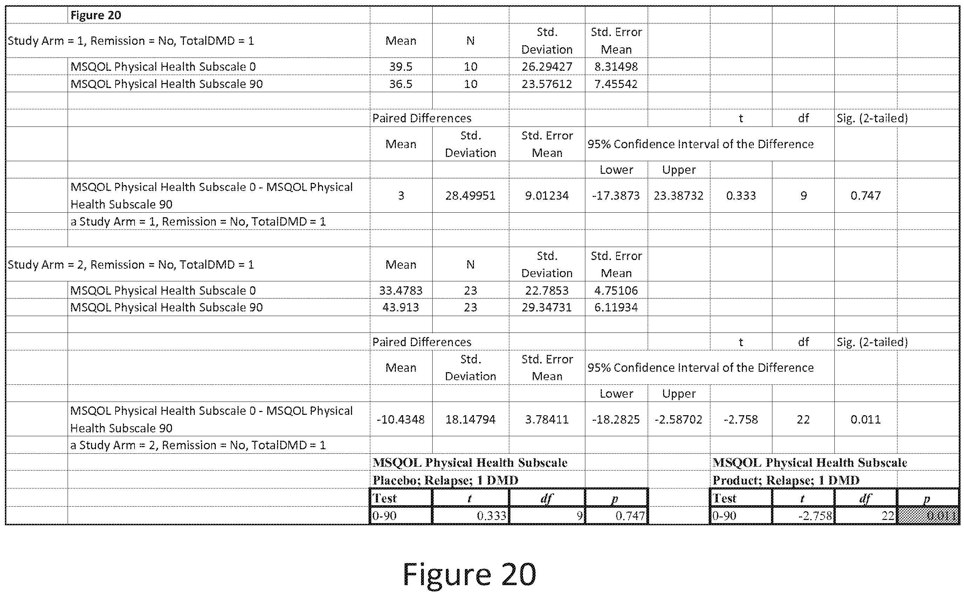

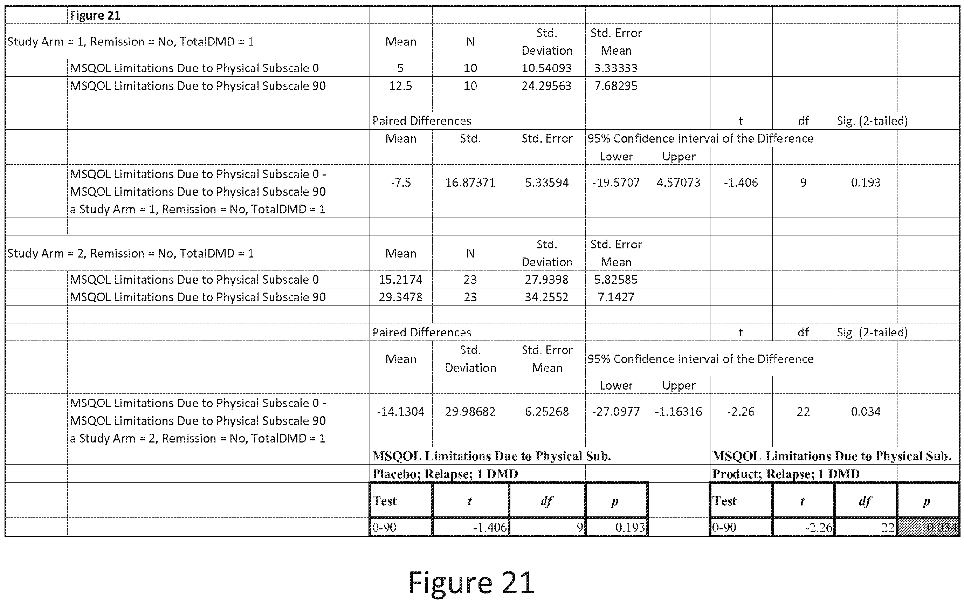

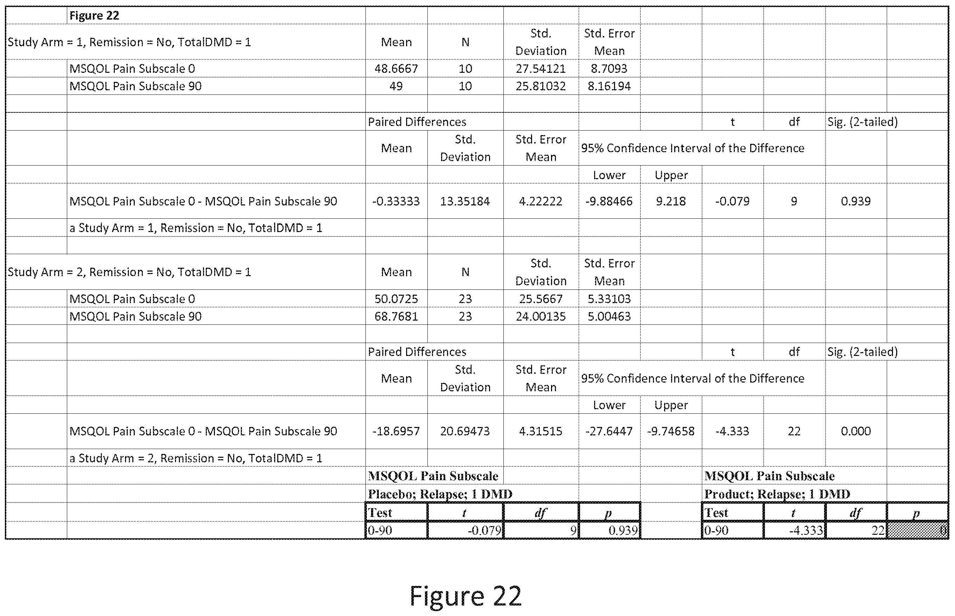

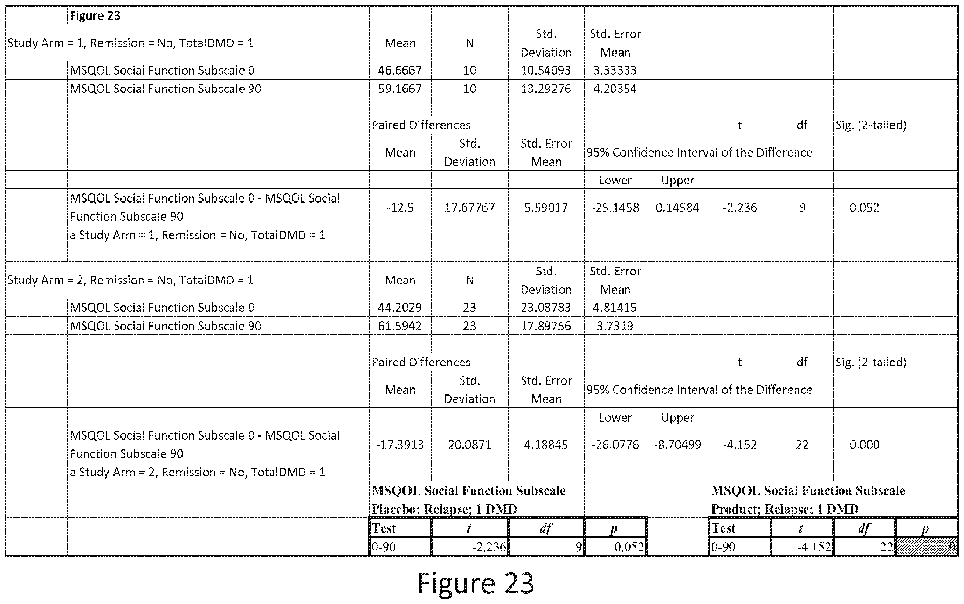

For individuals with RRMS who were in a relapse state and taking DMDs statistically significant improvements were seen in a number of different measures. The MSIS scores for the apoaequorin arm showed a 13.46% greater improvement than the placebo arm (Figure 14). The MFIS Composite scores for the apoaequorin arm showed a 24.8% improvement, while the placebo arm recorded an 11.57% improvement (Day 90 vs. Day 0) (Figure 15). The MFIS Physical Health subscale for the apoaequorin arm at Day 90 was 25.36% better than Day 0, while the placebo arm reported an 11.69% improvement over the same period (Figure 16). On the MFIS Cognitive subscale, the apoaequorin arm showed a greater improvement than the placebo arm (23% vs. 20.12%) (Figure 17). The MFIS Psychosocial subscale showed improvements in the apoaequorin arm that was 50% larger than that seen in the placebo arm (Figure 18). All apoaequorin values were statistically significant at p<0.05 while none of the placebo values were statistically significant. Statistically significant improvements were seen in the apoaequorin arm, but not in the placebo arm, for the MSQOL Physical Health Composite scores (31.56% vs. 3.58%) (Figure 19), the MSQOL Physical Health subscale (31.17% vs. -7.59%) (Figure 20), the MSQOL Role Limitations-Physical subscale (Figure 21), the MSQOL Pain subscale (37.32% vs. 0.68%) (Figure 22), and the MSQOL Social Function subscale (39.34% vs. 26.78%) (Figure 23).

For those individuals with RRMS and SPMS, a statistically significant difference between apoaequorin and placebo was seen on the MSQOL Physical Health subscale and the MSQOL Health Perceptions subscale. A statistically significant difference was seen on the MSQOL Pain subscale and the MSQOL Energy subscale at Day 90.

For the population of individuals with RRMS and SPMS, a statistically significant difference was seen between individuals taking apoaequorin and those taking placebo without any DMDs. A statistically significant difference was seen in the MSIS Composite score, MSQOL Energy subscale, MSQOL Cognitive Function subscale, and the MSQOL Overall QOL Composite score.

For individuals with RRMS and SPMS and taking a DMD, a statistically significant difference was seen between individuals taking apoaequorin and those taking placebo on the MSQOL Physical Health subscale and the MSQOL Role Limitations-Physical subscale. A statistically significant difference was also seen on the MSQOL Energy subscale.

For the subset of individuals with RRMS, who were in a state of relapse prior to initiating apoaequorin, a statistically significant difference was seen between individuals receiving apoaequorin and those receiving placebo in the MSIS composite, MFIS Cognitive subscale, the MSQOL Physical Health, MSQOL Role Limitations-Emotional subscale, MSQOL Role Limitations-Physical subscale, MSQOL Pain subscale, MSQOL Energy subscale, MSQOL Social Function subscale, MSQOL Cognitive Function subscale, and the MSQOL Health Distress subscale.

For individuals with RRMS, who were in a state of remission prior to beginning the study, the apoaequorin arm showed 11.6% improvement from Day 0 to Day 90 in their MSIS Composite score (p=0.000) compared to a 4.5 decrease in the placebo arm. For these individuals a statistically significant difference was also seen between the apoaequorin and placebo arms on the MSQOL Physical Health subscale.

For those individuals with RRMS, who were not in a state of remission prior to beginning the study, the apoaequorin arm showed 21% greater improvement (Day 90 vs. Day 0) compared the placebo arm(Day 90 vs. Day 0). The difference between the Day 90 and the Day 0 apoaequorin scores were close significant (p=.070) (p=.070) at Day 90 and extremely significant (P<0.05) at all other time points. For this group the MFIS Physical subscale, MFIS Cognitive subscale, MSQOL Physical Health subscale, MSQOL Mental Health Composite subscale, MSQOL Cognitive Function subscale all showed improvements for the apoaequorin arm compared to the placebo arm which were statistically significant at most time points.

## Claims

1. Apoaequorin for use in alleviating a symptom associated with Multiple Sclerosis in a subject,
wherein the apoaequorin is formulated as an oral dose comprising the apoaequorin and a carrier,
wherein 40 mg to 80 mg of the apoaequorin is contained in said oral dose,
wherein preferably about 40 mg of the apoaequorin is contained in said oral dose.

2. The apoaequorin for use according to claim 1, wherein the symptom associated with Multiple Sclerosis is reduced quality of sleep, reduced energy, reduced mood, reduced cognitive function, or pain.

## Patentansprüche

1. Apoaequorin zur Verwendung bei der Linderung eines mit multipler Sklerose in Zusammenhang stehenden Symptoms bei einem Patienten,
wobei das Apoaequorin als eine orale Dosis mit dem Apoaequorin und einem Trägerstoff formuliert ist,
wobei 40 mg bis 80 mg des Apoaequorins in der oralen Dosis enthalten ist, wobei bevorzugt etwa 40 mg des Apoaequorins in der oralen Dosis enthalten ist.

2. Apoaequorin zur Verwendung nach Anspruch 1, wobei das mit multipler Sklerose in Zusammenhang stehende Symptom eine verminderte Schlafqualität, verringerte Energie, betrübte Stimmung, eingeschränkte kognitive Funktionen oder Schmerz ist.

## Revendications

1. Apoaéquorine pour l'usage dans le soulagement d'un symptôme associé à la sclérose en plaques chez un sujet,
dans laquelle l'apoaéquorine est formulée comme dosage oral comprenant l'apoaéquorine et un support pharmaceutique,
dans laquelle 40 mg à 80 mg d'apoaéquorine est contenu dans ledit dosage oral, dans laquelle de préférence 40 mg d'apoaéquorine est contenu dans ledit dosage oral.

2. Apoaéquorine pour l'usage selon la revendication 1,
dans laquelle le symptôme associé à la sclérose en plaques est la diminution de la qualité du sommeil, l'énergie diminuée, la diminution de l'humeur, la dégradation des fonctions cognitives ou de la douleur.
